# EUROPEAN PATENT APPLICATION

(11) **EP 1 529 784 A1**
(43) Date of publication of application: **11.05.2005**
(21) Application number: 04026250.3
(22) Date of filing: 05.11.2004
(51) Int. Cl.: C07K 14/38, A61K 31/20, A61K 31/201, A61K 31/325, A61K 33/02, A61K 33/06, A61K 35/70, C12P 1/02, C12P 21/00, C12R 1/69

(54) **Polymeric anhydride of magnesium and proteic ammonium phospholinoleate-palmitoleate.**

(30) Priority: 06.11.2003 BR 0305373
(71) Applicant: Iseu da Silva Nunes, Bosque Campinas SP 13026-121 (BR)
(72) Inventor: Da Silva Nunes, Iseu, Bosque Campinas SP (BR); Zenker Justo, Giselle, Cambui Campinas SP (BR); Duran Caballero, Nelson Eduardo, Casa 69 Betel Paulinia SP (BR)
(74) Representative: Duraes Rocha, Manuel

(57) **Abstract**

Protein Aggregate Magnesium-Ammonium Phospholinoleate-Palmitoleate Anhydride Immunomodulator, its Production Process and Formulation characterized in that an immunomodulator consisting of a protein aggregate of ammonium and magnesium phospholinoleate-palmitoleate anhydride, containing 11.6 ± 4.0% of total lipids, (22.7 ± 5.0% of palmitoleic acid, 42.9 ± 2.0% of linoleic acid, and 32.0 ± 3.0% of oxidated linoleic acid), 20.1 ± 0.9% of magnesium ions, 10.0 ± 3.3% of ammonium ions, 45.2 ± 2.7% of phosphate, and 0.49 ± 0.01% of protein (Asp 7.19%, Thr 3.56%, Ser 7.56%, Glu 8.53%, Pro 0.5%, Gly 9.69%, Ala 7.46%, Val 1.0%, Met 4.38%, Isoleu 2.54%, Leu 3.03%, Tyr 0.5%, Phe 1.0%, His 2.83%, Lys 3.56%, Trp 1.3%, and Arg 35.2%). This compound is produced by means of the *Aspergillus oryzae* fungus, cultured in a culture medium consisting of an aqueous solution of oat and gelatin in the proportion of 10:1 oat:gelatin in weight for a period of 5 days in a bioreactor kept between 20° and 35° C, with pH stabilized in the range of 2 to 4, under low aeration (-2 liters/min) and slow agitation (5 rotations per hour), followed by mechanical filtration of the culture medium; extraction of the compound with ethyl acetate; precipitation of the compound under pH 11 by a 20% aqueous solution of sodium carbonate; washing of the crystals in ethyl acetate and ether; and drying. For use in biological systems, the compound is formulated in injectable form or in the form of polymeric microspheres. The injectable form is obtained by dissolving the compound in a phosphate-saline buffer solution (PBS) 0.1 M, and is stable for 30 days when kept between 5°C and 8°C. The form of polymeric microspheres is achieved by preparing an emulsion containing the compound, obtained by solvent evaporation, using a biodegradable polymer, e.g. poly(epsilon-caprolactone), and poly(vinyl alcohol) (PVA) as a surfactant agent.

## Description

The present invention concerns a new chemical compound of protein aggregate of ammonium and magnesium phospholinoleate-palmitoleate anhydride, possessing as its main characteristic an immunomodulatory activity that confers it antitumoral, antiviral and antibacterial properties, and opens a broad field of application, in view of the importance of the host defense system response in these pathologies.

The second object of the present invention is an optimized production method for obtaining this compound.

Finally, as further objects of the present invention, two methods are presented to formulate the compound, in order to make it available for usage in biological systems.

It is generally known that immune system modulation is an alternative therapeutic strategy or an adjuvant to conventional chemotherapy, particularly in situations of deficient immune response. This is one possible application of the compound of the present invention.

Another field of application that can be cited as example is the prophylaxis of infections in risk situations such as in hospitalized patients, or in patients whose immune response is deficient or has been diminished for any reason.

Finally, the emergence of new types of viruses, as well as the appearance of more aggressive mutations of known viruses and their rapid global dissemination, further compounded by the difficulty in developing vaccines in a short time, makes it highly desirable to develop substances capable of modifying or modulating the immune response, to be used either as monotherapies or adjuvant therapies in association with other pharmaceuticals and vaccines.

From these few considerations it is evident that new compounds with immunomodulatory properties, capable of modifying the effectiveness of the biological responses of the organism, can be highly useful and have a broad range of application.

However, the majority of compounds developed till the present state of the art, and used in the treatment of viral and bacterial pathologies, as well as the compounds used in the treatment of neoplasias, are plagued by considerable problems in their practical utilization, be through problems in the use of therapeutics caused by toxicity problems, such as chemotherapeutics case, used in the treatment of neoplasias or antibacterials, that have its efficaciousness compromised along of the using time, by the appearance of varieties of resistant microorganisms.

Medicine relatively recent progress compounds, with recognized immunomodulatory activity, such as interferon's, by various factors, in between, toxicity and high treatment cost, reveals in the practical therapeutic, of restrict usefulness, being its use restrict to a little applications. This demonstrates that the field of development for new compounds with immunodulatory activity is very extensive, since although the immunotherapy is promising as a therapeutic strategy, a few immunomodulatory compounds are currently in use.

In a patent that can be cited as a previous invention (Nunes & Duran, US Patent 5,073,630 (1991), European Patent Application EP 0 400 477 A1 (1991)), some biological purposes, such as antitumoral and antiviral effects, were described. Still in this compound cited (US-Patent 5,073,630, European Patent Application EP 400,477(1991)), a limited in vitro immunostimulant effect was observed, only when used in mouse lymphocyte spleen cultures that have had their mitogenesis accelerated. This effect, being limited, would not allow suggesting the practical usefulness of the cited compound as preference, such us an immunomodulator.

Meanwhile, biological results presented by the previous cited compound has allowed to visualize that the changes in the molecular composition of the previous cited compound, for example, associating new compounds, would allow permitting that a new and distinct product with immunomodulatory capacity be obtained, that what it was realized in the compound described in the title of the present invention, that presents extensive immunomodulatory properties, which do not occurs with one cited as a prior invention. The present invention is further distinguished by the presence of compounds distinct in its chemical composition and consequently presenting different structural formula in relation to the prior cited invention, as demonstrated in the present report.

The object of the present invention contains the above-mentioned parameters and said object could be defined as follows: invention of a protein family of ammonium and magnesium anhydride of phospholinoleate-palmitoleate having as a principal characteristic an extensive immunodulatory capacity, which allows to indicate its using as a compound to be used in antitumoral, antiviral and antibacterial therapy, as it has been described in the present application, as an exemplification, through several types of analysis with biological systems and even in experimental animals.

The starting point for the development of such a compound, with the above-mentioned attributes, were the biological properties of the individual components of the compound, as described in the literature:

### A) Magnesium

Biological properties: Magnesium *in vitro* which stimulates the production of T lymphocytes, whereas the Mg⁺² deficiency causes a dramatic reduction in the primary and secondary immune response, this activity being measured by the number of AFC of mice hypophysis C 57 B 1/6. The Mg⁺² are involved in the binding of the IgM complex with mouse lymphocytes. Magnesium ions stimulate apoptosis in the physiologic area of 0.8-1.2 mM. The results showing the caspase activation in the transduction of the magnesium-induced apoptotic signal (Black et al., Obstetrics & Gynecology 98, 319 (2001)).

### B) Linoleic acid

Biological properties: When it has been studied the linoleic acid influence added in the chicken diet, in the metabolic and physiologic response, following to a stimulation of the animals immune system, induced by lypopolysaccharides, it has been shown that the addition of linoleic acid in its diet, produces modulation preventing the manifestation of undesirable metabolic and physiologic effects, in relation to the animals control Takahashi et al., Br. Poultry Sci. 43, 47 (2002)).

### C) Palmitoleic acid

Biological properties: In a study of a culture of pancreatic islets of rats previously treated with saturated fatty acids, such as palmitic acid, it has been shown that palmitoleic acid is able to induce modulation consisting of improvement of insulin secretion by Beta cells. In contrast, the controls displayed a reduction of the secretion activity due to the action of palmitic acid (Maedler et al., Diabetes 50, 69 (2001)).

### D) Arginine

Biological properties: It is known that arginine induces apoptosis in culture cells. The proposed mechanism begins with activation of caspase-8, followed by release of Cytochrome-C and subsequently activation of downstream caspase-9 and caspase-3 to poly cleavage ADP-ribose) polymerase (Singh et al., J. Biol. Chem. 277, 37630 (2002)). It has been found that the presence of arginine in association with unsaturated fatty acids, combination that exists in the compound of the present invention, has been shown extremely efficient in oral infection prophylaxis, when this association is previously used before surgery, improving the immunometabolic response and decreasing the infection rate (Braga et al., Surgery 132, 805 (2002)). Arginine in concentration of 6 mg/100 ml in the diet allows a reduction of metastasis rate in Walker 256 tumor (Novaes et al., Arch. Latinoamer. Nutrition 50, 230 (2000)). Arginine plays an important role in the treatment of oesophageal carcinoma (Qin et al., Zhongguo Zhongliu Linchang 27, 591 (2000)). Polyarginine in injectable form is used as a booster causing no antibody response, making it an important component in building molecules with immunomodulatory properties for cancer treatment (Mattner et al., Cancer Res. 62, 1477 (2002)).

In the present invention these isolated chemical components were brought together into an integrated aggregate structure, with a synergistic biological action, and a compound was achieved with marked immunomodulatory activity, distinct from and wider than the biological properties of the isolated components as described in the literature.

The aggregate structure in the title was biologically obtained by means of a suitable fungus grown in a culture medium specially selected for being rich in the chemical components mentioned above, and for having the desired biological properties, in particular its immunomodulating properties. For instance, oat, one of the components used in the production process of the compound of the present invention, has high magnesium and phosphor content, as well as a considerable concentration of fatty acid, another component deemed desirable in the molecule of the present invention. Likewise, gelatin, another component in the production process of the present invention, was selected because it is a primary source of aminoacids such as arginine, which is particularly important because it is known to possess immunomodulatory properties.

After studies and experimentation with several species of fungi theoretically expected to produce the compound described in the present invention, the mycelium of the *Aspergillums oryzae* fungus was selected. This species is reported in the literature as being able to produce substances with therapeutic activity when cultured in cereals (Katyama et al., Oncology Reports 9(4) 817-822 Jul-Aug 2002). This production occurs when the *Aspergillums oryzae* mycelium is grown in a culture medium formulated to contain the chemical components that should be incorporated into the final structure of the compound described in the present invention.

In other words, the compound of the present invention is obtained as a result of biological action exerted by the mycelium of the *Aspergillums oryzae* fungus, which was selected to produce the aggregate structure described in the title when grown in a culture medium so formulated as to contain the individual chemical components of interest for the final product.

This culture medium, specifically formulated to obtain the title product by biological action of the *Aspergillums oryzae* fungus, consists of a mixture of oat and gelatin in the proportion of 10:1 oat: gelatin in weight. After 5 days of culture of the *Aspergillums oryzae* fungus in a bioreactor containing the above-mentioned culture medium, with temperature kept in the range of 20-35 C, pH kept in the range of 2-4, low aeration (~2 1/min) and low agitation (5 rotations/h), the culture medium, after undergoing the action of the *Aspergillums oryzae* fungus for the period of time mentioned above, is removed from the bioreactor, filtered and extracted with ethyl acetate, which finally allows to obtain the compound of the present invention request by precipitation under pH 11 by means a of solution of 20% sodium carbonate in water. The crystals are then washed in ethyl acetate and ether, and dried. The compound of the present invention is in crystalline form and has a high melting point (above 330° C).

The average molecular mass is 320000 Da and was determined by filtration in Sephacryl S-300 using Tris 0.02 M, pH 8.0. The chemical analysis of the compound of the present invention show the presence of 11.6 ± 4.0% of total lipids, (22.7 ± 5.0% of palmitoleic acid, 42.9 ± 2.0% of linoleic acid and 32.0 ± 3.0% of oxidized linoleic acid), 20.1 ± 0.9% of Mg ions, 10.0 ± 3.3% of ammonium ions, 45.2 ± 2.7% of phosphate, and 0.49 ± 0.01% of proteins. Protein aminoacids are distributed as follows: Asp 7.19%; Thr 3.56%; Ser 7.56%; Glu 8.53%; Pro 0.5%; Gly 9.69%; Ala 7.46%; Val 1.0%; Met 4.38%, Isoleu 2.54%, Leu 3.03%, Tyr 0.5%, Phe 1.0%, His 2.83%; Lys 3.56%, Trp 1.3%, and Arg 35.2%.

For use in injectable form, the compound of the present invention is formulated in a phosphate-saline buffer solution (PBS) 0.1 M. Solutions are stable for a period of up to 30 days if kept between 5°C and 8°C. For oral administration, the compound of the present invention is suspended in isotonic solutions.

The compound of this invention differs substantially, both in chemical characteristics and in biological properties, from the patented compound (US Patent 5,073,630 (1991), European Patent Application EP 0 400 477 A1 (1991)), that can be cited as previous invention, as shall be explained in the present report.

The difference in chemical composition between the compound of the present invention and the compound cited as previous invention (US Patent 5,073,630 (1991), European Patent Application EP 0 400 477 A1 (1991)) is the presence of palmitoleic acid in the molecule of the present invention, and its absence in the previous invention.

Biologically, the difference between the compound of the present invention and the previous invention is its broad immunomodulatory activity, prominent in this invention, and absent from the previous one. These marked immunomodulatory properties result from the presence of new components in its formula, such as the palmitoleic acid, absent from the previous invention, and from its different molecular structure.

The production method of the compound of the present invention also differs widely from that of the previous invention.

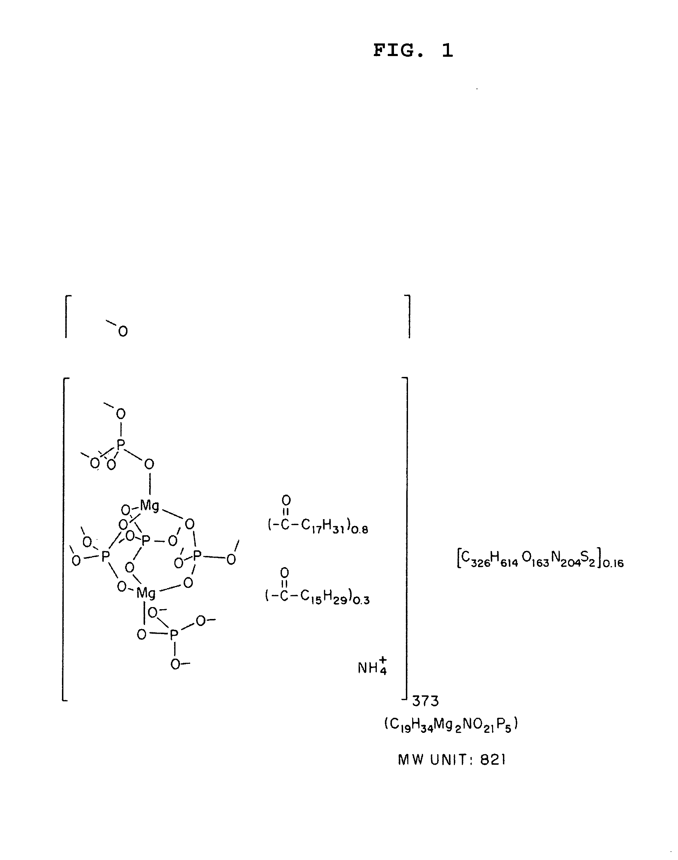

The chemical structure of the compound of the present invention was investigated by scanning electron microscopy, x-ray spectroscopy, infrared, and ultraviolet-visible spectroscopy, and its proposed structural formula is shown in Figure 1.

In order to exhibit the potential of the present invention, examples are given below. It should be pointed out that the scope of application of the invention is in no way limited to, or restricted by, these examples.
a) Antiviral properties through immune response modulation. In vivo studies have shown that treatment with the compound of the present invention promote a higher recovery rate of dogs carrying parvovirus and distemper virus. Blood of those animals treated with the compound of the present invention displayed a significant increase in white blood cell count, as compared to blood collected from control animals carrying the same infections but treated with only saline solution. This suggests an activation of defense mechanisms mediated by the immune system, which can be attributed to the compound of the present invention, as will be demonstrated in more detail by other animal experiments in the present report.
b) Antitumoral properties through immune response modulation. Intraperitoneal administration of 3 doses per week of 5.0 mg/kg of the compound of the present invention for a period of 7 weeks caused a significant decrease in the tumor growth rate and increased by 50% the survival of Balb/c mice which had been subcutaneously inoculated with plasmacytoma, and of WAB/Not mice which had spontaneous mammary carcinoma transplanted into subcutaneous tissue.
   A tumor regression was observed in 50% of female Wistar rats inoculated with Walker 256 tumor cells into subcutaneous tissue, and treated with daily subcutaneous doses of 0.5 mg/kg of the compound of the present invention.
   Treatment with doses of 5.0 mg/kg of the compound of the present invention, subcutaneously administered for a period of 7 days to Balb/c mice which had been intraperitoneally inoculated with Ehrlich ascetic tumor inhibited 50% of the tumor growth and increased by 50% the survival of the animals.
c) Immunomodulatory properties. Wistar female rats subjected to severe immune system depression by exposure to 4 Gy of gamma radiation (source: cobalt bomb), and inoculated with Walker 256 tumor cells in the subcutaneous tissue did not respond to treatment with subcutaneous administration of the compound of the present invention, suggesting that the compound acts on the host defense mechanisms and that these are fundamental for its action.
   A dose-dependent increase was observed in the number of granulocyte-macrophage precursor cells in the bone marrow (CFU-GM) of normal Balb/c mice intraperitoneally inoculated with Ehrlich ascetic tumor when the animals were subcutaneously treated for 7 days with doses between 0.5 a 5.0 mg/kg of the compound of the present invention. The 5.0 mg/kg dosage administered for 7 days before or after inoculation proved particularly efficient in myelopoiesis modulation in mice with the tumor. Similarly, the same treatment protocol promoted a significant reduction of CFU-GM count in the spleen and splenomegalia of animals inoculated with Ehrlich ascetic tumor. The compound of the present invention did not present in vitro effects on the growth and differentiation of granulocyte-macrophage precursor cells in the bone marrow from normal donor Balb/c mice.
   These findings clearly indicate that the compound of the present invention promotes the immune mechanisms that regulate bone marrow precursor cells, and that are involved in controlling the changes caused by the tumor in the host hematolymphopoietic system.
   Pre-treatment with the compound of the present invention, subcutaneously administered for 7 days, also promoted myeloprotection in Balb/c mice intraperitoneally inoculated with the bacterium *Lysteria monocytogenes,* known to cause lysteriosis; dosages of 0.5 and 5.0 mg/kg protected 30% and 40% of the mice, respectively, against lethal infection caused by this bacterium. The fact that the compound of the present invention does not display in vitro antibacterial activity indicates that the protective effects observed in the lysteriosis model are mediated by the immune system.
   Treatment of Balb/c mice, inoculated with Ehrlich ascetic tumor, with 5.0 mg/kg of the compound of the present invention for a period of 7 days was also able to modulate the functional ability of mature immunocompetent cells, supplying additional evidence about the action mechanisms of the compound. This treatment protocol stimulated the proliferative ability of lymphocytes and the cytotoxicity mediated by natural killer (NK) cells.
   Additionally, a modulation of the cytokines which are regulatory of effector events of the immunological system was observed in these mice. A significant increase in interleukin-2 (IL-2) and gamma-interferon (IFN- ) was detected in cultures of splenic cells from these animals, stimulated by the mitogen concanavalin A (ConA). No alteration was observed in interleukin-4 (IL-4) levels, and production of interleukin-10 (IL-10) in mice with tumors was significantly reduced by treatment with this compound.
   Levels of the same cytokines found in Balb/c mice infected with L. monocytogenes and subjected to pre-treatment with 5.0 mg/kg of the compound of the present invention supplied additional information about its biological activity. The increase in production of IL-2 e IFN- by the culture of spleen cells from these animals indicate a modulation of the inflammatory-like response, which is fundamental for eradication of the infection.
   Taken together, the above results indicate that the compound of the present invention directly or indirectly modulates the activity of immunocompetent cells, and displays antitumoral, antiviral e antibacterial effects through immunotherapeutic mechanisms. In other words, the compound of the present invention unequivocally possesses extensive immunomodulatory action.
d) Cytotoxic properties. Cytotoxicity of the compound of the present invention was studied in the range of 10⁻⁴ to 10⁻⁸ M in 53 tumor cell cultures representing 8 distinct tumor types (lung, colon, central nervous system, melanoma, ovary, kidney, prostate and mammary) and leukemia. The compound of the present invention did not display cytotoxicity for these tumor cells. No cytogenic alterations in lymphocytes for doses up to 100 µg/ml were observed either.
e) *In vivo* toxicological properties. No toxic effects were observed in acute toxicity experiments in mice (single doses up to 2000 mg/kg), in tufted capuchin monkey (*Cebus apella*) (30 days, doses up to 30 mg/kg), and Wistar rats (90 days, doses up to 100 mg/kg). No significant variation was found in hematological, biochemical, physiological and anatomopathological parameters in animals treated with the compound of the present invention as compared to control animals.
f) Immunotoxic properties. No immunotoxic properties were found. In studies with tufted capuchin monkey (*Cebus apella*), an increase of total leucocytes was observed, particularly polymorph nuclear neutrophils, and a small increase in the lymphocyte population. With regard to hematological parameters, a minor decrease was observed for hematocrit and hemoglobin. The following organs were analyzed: bone marrow, spleen, lymphonodes, tonsils, and renal gland e Peyer's patch.
   In the bone marrow, a slight activation of the granulocytic series was observed. The spleen presented an increase of secondary follicles in the white pulp.
   Erytrophagocytosis was visible in the red pulp, often with the presence of granulocytes among reticular cells. However, these cell alterations, found in the anatomopathological examination of the spleen from animals treated with the compound of the present invention, cannot be considered pathological in character; rather, they are due to immune system modulation caused by the use of the compound of the present invention.
g) Embriotoxicity. For the evaluation of this parameter, doses of 1, 10 and 100 mg/kg were used. Groups of 10 Wistar rats (5 male and 5 female) were subcutaneously treated with the compound of the present invention daily for 13 weeks. After this period 5 couples per dosage group were kept together and received the compound throughout the 14th week. In the 15th week the males were sacrificed, and the females continued to receive daily doses till the 19th week, i.e., the period of gestation and lactation. After birth, from the first to the 12th day, the descendants had the following parameters evaluated: body weight, ocular diameter, thoracic perimeter, transverse, longitudinal and inter-auricular measurements. X-ray analysis was performed in one-third of the animals. After 12 days, and after blood and urine collection, the descendants were sacrificed and the following organs or tissues removed for anatomopathological study: spleen, thymus, liver, kidney and heart.
   Another 27 structures were also removed from males, females and descendants. Taken together, the results indicate that the compound of the present invention did not interfere with the reproduction of the treated females, nor did it induce teratogenic effects in the descendants.
h) Bioavailability. The bioavailability of the compound of the present invention was evaluated in Wistar rats by administering a single intraperitoneal dose of 750 mg/kg. The plasma pharmacokinetics followed the expected behavior, presenting a t_{½} of about 2h. Study of the organs showed an accumulation of magnesium, particularly in the spleen and pancreas, 1h after administration of the compound.

The observed increase in the spleen magnesium levels not only provides information about pharmacokinetics of the compound but also offers relevant data for understanding the immunomodulatory ability of the compound of the present invention, since this high concentration of magnesium observed in the spleen reflects its importance as the main lymphoid organ responsible for differentiation of defense cells that constitute the immune system.

Several studies report the importance of magnesium in reactions affecting immunocompetence, as for instance the phagocyte adherence and granulation processes. It is possible that these are being modulated by the compound of the present invention.

The above experiments, cited as exemplification, characterize the compound of the present invention as an original compound that, for its chemical properties and biological effects, has not been described in the state of the art until now. It possesses immunomodulatory ability as its main characteristic, which allows us to propose a broad range of practical applications as a therapy of viral and bacterial pathologies, as well as neoplastics of various kinds, either preventively or curatively, either in isolation or in combination with other pharmaceuticals and substances.

Toxicity tests of the compound of the present invention, carried out in several cellular systems and experimental animals, in different doses and exposure times, demonstrate that it can safely be proposed for clinical use, as no noteworthy side effects have been detected that could prevent its use or reduce its practical therapeutic usefulness.

The compound of the present invention can be formulated for usage in biological systems by two different methods. The first one, described in detail in Claim 3, for use in injectable form, dissolves the compound in a phosphate-saline buffer solution (PBS) 0.1 M. This solution is stable for 30 days if kept between 5 and 8 C.

The second method, described in detail in Claim 4, allows the compound of the present invention to be formulated in the form of polymeric micro spheres. The method involves preparation of an emulsion containing the compound, obtained by solvent evaporation, using a biodegradable polymer, e.g. poly(epsilon-caprolactone), and poly(vinyl alcohol) (PVA) as a surfactant agent. The proportion of polymer to surfactant is 1:5.

For the purpose of exemplification, the procedure described below indicates the amounts of solvent and other materials needed to prepare 6 mg of the compound of the present invention in the form of polymeric micro spheres.

Aqueous phase: In a 250 ml beaker, the surfactant agent (PVA poly(vinyl alcohol), concentration 5%) is dissolved in distilled water (80 ml) under mild mechanical agitation, avoiding foam formation, until a solution is formed.

Organic phase: In a 50 ml Beaker, the polymer poly(epsilon-caprolactone) (concentration 1,3%) is dissolved in an appropriate mixture of organic solvents (2.5 ml of DMSO dimethylsulphoxide, and 5 ml of chloroform), along with 6 mg of the compound of the present invention.

The organic phase (solvent + polymer + compound of the present invention) is added drop by drop to the aqueous phase (surfactant PVA in 5% concentration) under agitation at 570 rpm.

After completing the addition, a two-phase system is formed (a turbid dispersion similar to oil in water). The system is left under agitation for 12h until the solvent evaporates.

After evaporation of all the solvent, the dispersion is placed in a tube and centrifuged to collect the micro particles. The precipitate is collected and redispersed in distilled water. The solution containing the precipitate is washed and centrifuged 3 times, and the micro particles collected.

The micro particles are redispersed in distilled water in a test tube. The dispersion is frozen inside the tube by means of an external bath of liquid nitrogen and transferred for lyophilization for 24h. A dry, fine powder is obtained, composed of polymeric micro spheres of the compound of the present invention.

## Claims

1. Protein Aggregate Magnesium-Ammonium Phospholinoleate-Palmitoleate Anhydride Immunomodulator, its Production Process and Formulation **characterized in that** an immunomodulator consisting of a protein aggregate of ammonium and magnesium phospholinoleate-palmitoleate anhydride, that in chemical analysis has shown the presence of 11.6 ± 4.0% of total lipids, (22.7 ± 5.0% of palmitoleic acid, 42.9 ± 2.0% of linoleic acid, and 32.0 ± 3.0% of oxidated linoleic acid), 20.1 ± 0.9% of magnesium ions, 10.0 ± 3.3% of ammonium ions, 45.2 ± 2.7% of phosphate, and 0.49 ± 0.01% of proteins. The aminoacid distribution in the protein is: Asp 7.19%, Thr 3.56%, Ser 7.56%, Glu 8.53%, Pro 0.5%, Gly 9.69%, Ala 7.46%, Val 1.0%, Met 4.38%, Isoleu 2.54%, Leu 3.03%, Tyr 0.5%, Phe 1.0%, His 2.83%, Lys 3.56%, Trp 1.3%, and Arg 35.2%.

2. Protein Aggregate Magnesium-Ammonium Phospholinoleate-Palmitoleate Anhydride Immunomodulator, its Production Process and Formulation **characterized in that** a production process for the protein aggregate of ammonium and magnesium phospholinoleate-palmitoleate anhydride (the compound in Claim 1), involving the following steps: culture of the *Aspergillums oryzae* fungus in a culture medium consisting of an aqueous solution of oat and gelatine in the proportion of 10:1 oat:gelatine in weight, for a period of 5 days in a bioreactor kept between 20° and 35° C, with pH stabilized in the range of 2 to 4, under low aeration (2 litters/min) and slow agitation (5 rotations per hour); mechanical filtration of the culture medium; extraction of the compound with ethyl acetate; precipitation of the compound under pH 11 by a 20% aqueous solution of sodium carbonate; washing of the crystals in ethyl acetate and ether; and drying.

3. Protein Aggregate Magnesium-Ammonium Phospholinoleate-Palmitoleate Anhydride Immunomodulator, its Production Process and Formulation **characterized in that** a formulation in injectable form for the protein aggregate of ammonium and magnesium phospholinoleate-palmitoleate anhydride (the compound in Claim 1), obtained by dissolving the compound in a phosphate-saline buffer solution (PBS) 0.1 M. The formulation is stable for 30 days when kept between 5°C and 8°C.

4. Protein Aggregate Magnesium-Ammonium Phospholinoleate-Palmitoleate Anhydride Immunomodulator, its Production Process and Formulation **Characterized in that** a formulation in polymeric micro spheres for the protein aggregate of ammonium and magnesium phospholinoleate-palmitoleate anhydride (the compound in Claim 1), achieved by preparing an emulsion containing the compound, obtained by solvent evaporation, using a biodegradable polymer, e.g. poly(epsilon-caprolactone), and poly(vinyl alcohol) (PVA) as a surfactant agent, in the proportion of 1:5 polymer: surfactant in weight. For the purpose of exemplification and in no way restricting the field of application of this claim, the procedure described below indicates the amounts of solvent and other materials needed to prepare 6 mg of the compound of the present invention in the form of polymeric micro spheres.
Aqueous phase: In a 250 ml Beaker, the surfactant agent (PVA, concentration 5%) is dissolved in distilled water (80 ml) under mild mechanical agitation, avoiding foam formation, until a solution is formed.
Organic phase: In a 50 ml beaker, the polymer poly(epsilon-caprolactone) (concentration 1,3%) is dissolved in an appropriate mixture of organic solvents (2.5 ml of DMSO and 5 ml of chloroform), along with 6 mg of the compound in Claim 1.
The organic phase (solvent + polymer + compound in Claim 1) is added drop by drop to the aqueous phase (surfactant PVA in 5% concentration) under agitation at 570 rpm.
After completing the addition, a two-phase system is formed (a turbid dispersion similar to oil in water). The system is left under agitation for 12h until the solvent evaporates.
After evaporation of all the solvent, the dispersion is placed in a tube and centrifuged to collect the micro particles. The precipitate is collected and redispersed in distilled water.
The solution containing the precipitate is washed and centrifuged 3 times, and the micro particles collected.
The micro particles are redispersed in distilled water in a test tube. The dispersion is frozen inside the tube by means of an external bath of liquid nitrogen and transferred for lyophilization for 24h. A dry, fine powder is obtained, composed of polymeric micro spheres of the compound of the present invention.
